# EUROPÄISCHE PATENTSCHRIFT

(11) **EP 1 382 691 B1**
(45) Veröffentlichungstag und Bekanntmachung des Hinweises auf die Patenterteilung: **18.10.2006**
(21) Anmeldenummer: 03015260.7
(22) Anmeldetag: 07.07.2003
(51) Int. Cl.: C12Q 1/37, A01N 61/00

(54) **Verfahren zur Isolation des 20S Proteasoms, und Verfahren zur Identifikation von Inhibitoren des 20S und 26S Proteasoms**
Method for the isolation of the 20S proteasome, and methods for the identification of 20S and 26S proteasome inhibitors
Procédé d'isolation du proteasome 20S, et procédé d'identification d'inhibiteurs des proteasomes 20S et 26S

(30) Priorität: 19.07.2002 DE 10232902
(43) Veröffentlichungstag der Anmeldung: 21.01.2004
(73) Patentinhaber: Bayer CropScience AG, 40789 Monheim (DE); Max-Planck-Gesellschaft zur Förderung der Wissenschaften e.V., 80539 München (DE)
(72) Erfinder: Aichinger, Christian, Dr., 81477 München (DE); Schreier, Peter, Prof.Dr., 50674 Köln (DE); Ebbert, Roland, Dr., 40789 Monheim (DE); Huber, Robert, Prof.Dr., 82110 Germering (DE); Groll, Michael, Dr., 94072 Bad Füssing (DE)

(56) Entgegenhaltungen:
- DO HEE LEE & ALFRED GOLDBERG: "Selective inhibition of the proteasome-dependent and vacuolar pathways of protein degradatin in Saccaromyces cerevisiae" JOURNAL OF BIOLOGICAL CHEMISTRY, AMERICAN SOCIETY OF BIOLOGICAL CHEMISTS, BALTIMORE, MD, US, Bd. 44, Nr. 271, 1996, Seiten 27280-27284, XP002078281 ISSN: 0021-9258
- JONES R W ET AL: "VARIATION IN SENSITIVITY AMONG ANASTOMOSIS GROUPS OF RHIZOCTONIA-SOLANI TO THE ANTIBIOTIC GLIOTOXIN" PLANT DISEASE, Bd. 71, Nr. 1, 1987, Seiten 34-36, XP009019179 ISSN: 0191-2917
- SAEKI YASUSHI ET AL: "Rapid isolation and characterization of the yeast proteasome regulatory complex" BIOCHEMICAL AND BIOPHYSICAL RESEARCH COMMUNICATIONS, Bd. 273, Nr. 2, 5. Juli 2000 (2000-07-05), Seiten 509-515, XP002268381 ISSN: 0006-291X
- EMMERLICH VERA ET AL: "Isolation and subunit composition of the 20S proteasome of Giardia lamblia" MOLECULAR AND BIOCHEMICAL PARASITOLOGY, Bd. 100, Nr. 1, 15. Mai 1999 (1999-05-15), Seiten 131-134, XP002268382 ISSN: 0166-6851
- HORI HIROSHI ET AL: "Isolation and characterization of two 20S proteasomes from the endoplasmic reticulum of rat liver microsomes" JOURNAL OF BIOCHEMISTRY (TOKYO), Bd. 126, Nr. 4, Oktober 1999 (1999-10), Seiten 722-730, XP009019175 ISSN: 0021-924X
- FURET P ET AL: "MODELING OF THE BINDING MODE OF A NON-COVALENT INHIBITOR OF THE 20S PROTEASOME. APPLICATIONS TO STRUCTURE-BASED ANALOGUE DESIGN" BIOORGANIC & MEDICINAL CHEMISTRY LETTERS, OXFORD, GB, Bd. 11, Nr. 10, 21. Mai 2001 (2001-05-21), Seiten 1321-1324, XP001059888 ISSN: 0960-894X
- KOGUCHI Y ET AL: "TMC-95A, B, C AND D, NOVEL PROTEASOME INHIBITORS PRODUCED BY APIOSPORA MONTAGNEI SACC. TC 1093 TAXONOMY, PRODUCTION, ISOLATION AND BIOLOGICAL ACTIVITIES" JOURNAL OF ANTIBIOTICS, JAPAN ANTIBIOTICS RESEARCH ASSOCIATION. TOKYO, JP, Bd. 53, Nr. 2, Februar 2000 (2000-02), Seiten 105-109, XP002953124 ISSN: 0021-8820

## Beschreibung

Die vorliegende Erfindung betrifft Verfahren zum Identifizieren von Fungiziden.

Unerwünschtes Pilzwachstum, dass z.B. in der Landwirtschaft jedes Jahr zu beträchtlichen Schäden führt, kann durch die Verwendung von Fungiziden kontrolliert werden. Die Ansprüche an Fungizide sind dabei hinsichtlich ihrer Wirksamkeit, Kosten und vor allem ihrer Umweltverträglichkeit stetig angestiegen. Es existiert deshalb ein Bedarf an neuen Substanzen bzw. Substanzklassen, die zu leistungsfähigen und umweltverträglichen neuen Fungiziden entwickelt werden können. Im Allgemeinen ist es üblich, in Gewächshaustests nach solchen neuen Leitstrukturen zu suchen. Solche Tests sind jedoch arbeitsintensiv und teuer. Die Anzahl der Substanzen, die im Gewächshaus getestet werden können, ist entsprechend begrenzt. Eine Alternative zu solchen Tests ist die Verwendung von sogenannten Hochdurchsatzverfahren (HTS = high throughput screening). Dabei werden in einem automatisierten Verfahren eine große Anzahl von Einzelsubstanzen hinsichtlich ihrer Wirkung auf Zellen, individuelle Genprodukte oder Gene getestet. Wird für bestimmte Substanzen eine Wirkung nachgewiesen, so können diese in herkömmlichen Screeningverfahren untersucht und gegebenenfalls weiter entwickelt werden.

Ideale Fungizide sind z.B. solche Stoffe, die Genprodukte hemmen, die eine entscheidende Bedeutung bei der Ausprägung der Pathogenität eines Pilzes haben. Ein Beispiel für ein solches Fungizid ist der Wirkstoff Carpropamid, der die Melaninbiosynthese des Pilzes hemmt und so die Ausprägung intakter Appressorien (Haftorgane) verhindert. Es gibt jedoch nur sehr wenige bekannte Genprodukte, die für Pilze eine solche Rolle spielen. Darüber hinaus sind Fungizide bekannt, die durch die Hemmung entsprechender Biosynthesewege zur Auxotrophie der Zielzellen und in der Folge zum Verlust der Pathogenität führen.

Ein anderer wichtiger Ansatzpunkt sind Polypeptide oder Polypeptidkomplexe, die eine zentrale Rolle in essentiellen zellulären Prozessen oder essentiellen Proteinaktivitäten spielen. Im Vordergrund steht dabei die Hemmung von Enzymreaktionen, es kann aber auch die Interaktion von Proteinen oder das funktionelle Zusammenspiel komplexer Proteinmaschinerien gehemmt werden.

Ein Beispiel für eine solche komplexe "Proteinmaschinerie", die im Mittelpunkt zellulärer Abläufe steht, ist das 26S Proteasom. Die biologische Funktion des 26S Proteasoms umfasst dabei eine Vielzahl von Aufgaben, u. a. den Abbau fehlgefalteter und falsch assemblierter Proteine und Peptide, die Regulation der Stressantwort durch z.B. den Abbau von Transkriptionsfaktoren oder die Kontrolle des Zellzyklus durch den Abbau von Zyklinen. Für die Immunantwort der Zelle werden Peptide so prozessiert, dass sie anschließend vom MHC I Komplex an der Außenfläche präsentiert werden können. Das katalytische Kernstück des 26S Proteasoms ist das 20S Proteasom. Das 20S Proteasom selbst besitzt verschiedene, der enzymatischen Aktivität des 26S Proteasoms zugrunde liegende Peptidaseaktivitäten, weshalb insbesondere das 20S Proteasom als Zielort für Substanzen von Interesse ist, die diese enzymatischen Aktivitäten beeinflussen.

Die proteolytische Aktivität des Proteasoms *in vivo* benötigt die Ubiquitinierung der Zielproteine und verbraucht ATP. Für den Abbau der Zielproteine wird dabei das Ubiquitin auf das Zielprotein übertragen. Das ubiquitinierte Protein wird dann zum Proteasom gebracht wo es abgebaut wird und das Ubiquitin einem Recyling unterworfen wird.

Es ist bekannt, dass alle Proteine des 20S Proteasoms bis auf eines in *S. cerevisiae* für das Wachstum der Zelle notwendig sind. Der Komplex besitzt mehrere unterschiedliche proteolytische Aktivitäten, so z.B. eine Peptidyl-Glutamyl-Peptid hydrolysierende (PGPH) Aktivität, eine tryptische und eine chymotryptische Aktivität. Mutationsanalysen haben ergeben, dass die chymotryptische Aktivität die, wichtigste im Komplex ist. Das 20S Proteasom lässt sich bislang nicht heterolog exprimieren, die native Reinigung von aktiven 20S Proteasomen aus Hefe wurde aber bereits beschrieben.

Der Begriff "enzymatische Aktivität" des 20S oder 26S Proteasoms, wie er hierin verwendet wird, bezieht sich auf zumindest eine der verschiedenen enzymatischen Aktivitäten des 20S oder 26 S Proteasoms. Ein "enzymatisch aktives" 20S oder 26S Proteasom ist demnach noch in der Lage, zumindest eine der natürlicherweise vorhandenen enzymatischen Reaktionen auszuführen.

Das eukaryontische 26S Proteasom besteht aus je einem proteolytischen und zwei regulatorischen Komplexen. Das 20S Proteasom ist aus 7 verschiedenen α- und sieben verschiedenen β-Untereinheiten aufgebaut, die bereits aus verschiedenen Organismen kloniert und sequenziert wurden. Das 20S Proteasom aus der Bäckerhefe (*S. cerevisiae*) hat ein Molekulargewicht von ca. 700 kD. Die Reinigung von Proteasomen aus Eukaryonten ist ebenfalls schon in verschiedenen Dokumenten beschrieben worden (z. B. WO 98/42829 A1; EP 0 345 750 A2). Die Bemühungen, Proteasomen bestmöglich zu reinigen, resultierten schließlich auch in der Strukturaufklärung für das 20S Proteasome aus *S. cerevisiae* (WO 98/42829 A1; Groll et al. (1997), Structure of the 20S Proteasom from yeast at 2.4 Å resolution, *Nature 386,* 463-471).

In Coux et al. (1998), (Enzymes catalyzing ubiquitination and proteolytic processing of the p105 precursor of nuclear factor kappaB1. *J. Biol. Chem. 273*(15), 8820-8828), werden die multiplen proteolytischen Aktivitäten des 20S Proteasoms als chymotrypsinartig, trypsinartig, PGPH, verzweigte Aminosäureketten und kleine neutrale Aminosäuren vorziehend beschrieben. Die proteolytische Aktivität kann durch verschiedene Induktionsbedingungen verstärkt werden. Dazu zählt z.B. die Erhitzung des Proteasoms auf 55°C oder die Zugabe von SDS. Bekannt sind außerdem durch McCormack et al. (1998), *Biochemistry 37,* 7792-7800, verschiedene Substrate zum Nachweis der proteolytischen Aktivität des 20S Proteasoms, wie z.B. Suc-Leu-Leu-Val-Tyr-AMC, Z-Leu-Leu-Arg-AMC und Z-Leu-Leu-Glu-2NA, wobei Suc für N-succinyl, AMC für 7-Amino-methylcoumarin, Z für Carbobenzyloxy und 2NA für 2-Naphthylamin steht.

In öffentlich zugänglichen Datenbanken, wie MIPS ("munich information center for protein sequences") oder SGD ("saccharomyces genome database") werden bis auf wenige Ausnahmen alle Untereinheiten des 26S Proteasoms als essentiell beschrieben.

Bekannt ist auch die Existenz von verschiedenen reversiblen und nicht-reversiblen Inhibitoren für die enzymatische Aktivität von Proteasomen *in vitro.* So haben Klafky et al. (1995), *Neuroscience Letters 201,* 29-32, die Wirkung des Proteasomen-Inhibitors Calpain Inhibitor 1 auf die Sekretion des β-Amyloid-Peptids untersucht.

In Fenteany et al. (1995), *Science, 268,* 726-731, wird Lactacystin als Metabolit von Streptomyceten beschrieben, der als Zellzyklus-Inhibitor wirkt und bei Maus-Neuroblastomazellen zur Induktion des Neuritenwachstums führt. Zelluläres Target für diesen Inhibitor ist das 20S Proteasom.

In Kroll et al. (1999) *Chem. Biol. 6*, 889-698, wird ein pilzlicher Metabolit, Epipolythiodioxopiperazin (Gliotoxin) beschrieben, der u.a. die Antigen-Prozessierung in Säugerzellen unterdrückt. Wie die Autoren zeigen konnten, ist Gliotoxin ein nichtkompetetiver Inhibitor des 20S Proteasoms *in vitro.*

Mellgren (1997) *J. Biol. Chem. 272(47),* 29899-29903, beschreibt die inhibierende Wirkung verschiedener Peptidyl-Verbindungen auf das humane 20S Proteasom. In dieser Veröffentlichung wird weiter beschrieben, dass diese Substanzen in Konzentrationen von 200 µM keine Wirkung auf das Wachstum von Pilzzellen (*S. cerevisiae*) hatten. Lee und Goldberg (1998), (Proteasome inhibitors cause induction of Heat shock proteins and trehalose, which together confer thermotolerance in *Saccharomyces cerevisiae, Molecular and Cellular Biology 18,* 30-38), zeigen in Übereinstimmung dazu, dass pharmakologisch hochwirksame Proteasomen-Inhibitoren wie MG-132 (Z-Leu-Leu-Leu-CHO) keinen oder nur einen minimalen Effekt auf die Wachstumsrate von Hefezellen bei 30°C haben.

In Stack et al. (2000), *Nature Biotechnology 18,* 1298-1302, wird ein zelluläres Assaysystem zur Identifizierung von Inhibitoren des Proteasoms beschrieben und auf die mögliche Verwendung dieser Inhibitoren bei der Behandlung von Alzheimer- oder Parkinson-Erkrankungen hingewiesen. Dabei wird die Halbwertszeit von Proteinen verkürzt, indem Ubiquitin gentechnisch so verändert wird, dass die damit markierten Proteine destabilisiert werden. Verhindert ein Inhibitor die Proteindegradation, so kann dies anhand eines Reportergens bestimmt werden.

In WO 00/33654 A1 wird die Verwendung von Inhibitoren des Proteasoms zur Behandlung verschiedener humaner Erkrankungen beschrieben. Als Ansatzpunkt für die Wirkung der Inhibitoren wird hier die immunmodulierende Aktivität des 26S Proteasoms genannt.

Das Proteasom wird also bislang als Zielprotein (Target) für die Behandlung verschiedener Erkrankungen des menschlichen Organismus beschrieben. Ob auch das Proteasom aus Pilzen für Wirkstoffe zugänglich ist und durch diese inhibiert oder moduliert werden kann, und ob solche Wirkstoffe auch *in vivo* eingesetzt, d.h. als Fungizide verwendet werden können, ist bislang weder eingehend untersucht noch beschrieben worden. Mellgren (1997), *J. Biol. Chem. 272(47),* 29899-29903, ist nur zu entnehmen, dass die dort beschriebenen Inhibitoren des humanen Proteasoms in einer Konzentration von 200 µM keine inhibitorische Wirkung gegenüber Hefezellen aufwiesen. Lee und Goldberg (1998) zeigen weiterhin, dass pharmakologisch hochwirksame Proteasomen-Inhibitoren keinen oder nur einen minimalen Effekt auf die Wachstumsrate von Hefezellen haben.

Damit ist dem Stand der Technik nur zu entnehmen, dass zwar Inhibitoren des humanen Proteasoms existieren, diese jedoch bei Pilzen keine Wirkung zeigen. Das pilzliche Proteasom scheint also der inhibitorischen Wirkung der Verbindungen nicht zugänglich zu sein. Gleichwohl wäre es wünschenswert, neue Fungizide zur Verfügung zu stellen, die neue Wirkungsorte und Mechanismen haben, um damit z.B. einer Resistenzbildung gegenüber bekannten Fungiziden mit anderen Wirkorten vorzubeugen, und um wirksamere oder spezifischere, und damit auch umweltverträglichere Fungizide zu entwickeln.

Aufgabe der vorliegenden Erfindung war es deshalb, ein neues Target aus Pilzen für die Identifizierung von Fungiziden zur Verfügung zu stellen.

Im Rahmen der vorliegenden Erfindung wurde nun überraschenderweise gefunden, dass das bislang im Zusammenhang mit der Entwicklung von neuen Fungiziden als ungeeignet bekannte pilzliche 26S bzw. 20S Proteasom trotz der Erkenntnisse von Mellgren (1997) und Lee und Goldberg (1998) ein wirkungsvolles Zielprotein für spezifische Inhibitoren darstellt. Es wurde weiter gefunden, dass solche Inhibitoren in geeigneten Verfahren identifiziert und die mit diesen Verfahren gefundenen Inhibitoren überraschenderweise auch als Fungizide eingesetzt werden können. Dabei wurde auch festgestellt, dass auch bereits bekannte. Inhibitoren des humanen oder tierischen Proteasoms als Fungizide bzw. zur Herstellung eines Arzneimittels zur Behandlung von Pilzbefall bei Menschen oder Tieren verwendet werden können.

Im Rahmen der vorliegenden Erfindung wurde damit gefunden, dass pilzliche 26S bzw. 20S Proteasomen *in vitro* durch Wirkstoffe inhibiert werden können, und dass ein mit diesen Wirkstoffen behandelter pilzlicher Organismus durch die Behandlung mit diesen Wirkstoffen geschädigt und abgetötet werden kann. Die Inhibitoren von 26S/20S Proteasomen aus Pilzen können daher als Antimycotika und im Besonderen auch als Fungizide im Pflanzenschutz verwendet werden. In der vorliegenden Erfindung wird beispielsweise gezeigt, dass die Hemmung von 20S Proteasomen aus *S. cerevisiae* (Bsp. 1) ebenso gelingt wie die Hemmung von 20S Proteasomen aus *U. maydis* und *B. cinerea,* beides pflanzenpathogene Pilze (Bsp. 2). Die Behandlung dieser Pilze mit den in erfindungsgemäßen Testverfahren identifizierten Substanzen sowie mit bekannten Proteasomen-Inhibitoren führt zum Absterben der Pilze in synthetischen Medien bzw. auf der Pflanze.

20S Proteasomen aus Pilzen können daher zum Identifizieren von Fungiziden verwendet werden.

Der Begriff "Fungizid(e)", wie er weiter hierin verwendet wird, umfasst sowohl im Pflanzenschutz verwendete Stoffe, die zur Bekämpfung von pflanzenpathogenen Pilzen eingesetzt werden, als auch auf Stoffe, die zur Bekämpfung human- oder tierpathogener Pilze verwendet werden (Antimycotika).

"Inhibitoren" können kleine organisch-chemische Moleküle, Peptide oder Antikörper sein, die an die 20S oder an die größeren 26S Polypeptidkomplexe binden bzw. deren Aktivität beeinflussen. Weiterhin können Inhibitoren kleine organisch-chemische Moleküle, Peptide oder Antikörper sein, die an ein Molekül binden, welches wiederum an die erfindungsgemäßen Polypeptide bindet, und dadurch deren biologische Aktivität beeinflusst. Inhibitoren können natürliche Substrate und Liganden darstellen oder strukturelle oder funktionelle Mimetika davon. Bevorzugt handelt es sich beim Ausdruck "Inhibitor", wie er hierin verwendet wird, jedoch um solche Moleküle, die nicht die natürlichen Substrate bzw. Liganden darstellen. Der Begriff "Inhibitoren des 26S und/oder 20S Proteasoms" wie er hier verwendet wird, bezieht sich auf Inhibitoren, die in der Lage sind, eine oder mehrere enzymatische Aktivitäten des Proteasoms spezifisch zu inhibieren.

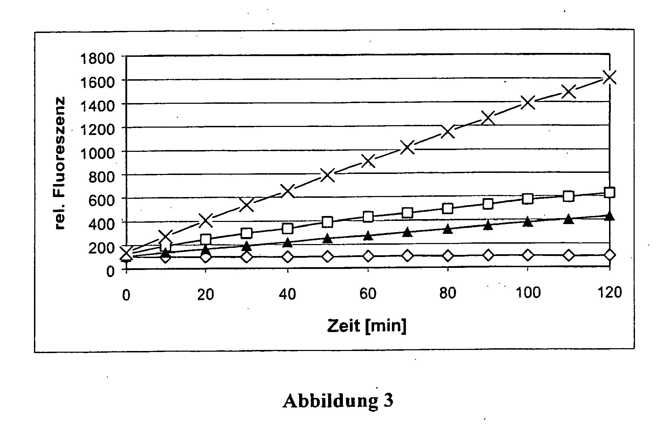

Es ist möglich, zur Identifizierung von Fungiziden 20S Proteasomen aus verschieden Pilzspezies zu verwenden, wie sie z.B. in der nachfolgenden Aufzählung genannt werden. So können beispielsweise 20S Proteasomen aus *S. cerevisiae* in einem erfindungsgemäßen Testverfahren zur Identifizierung von Fungiziden ebenso verwendet werden (Bsp. 1, Abb. 1) wie 20S Proteasomen aus den pflanzenpathogenen Pilzen *U. maydis* (Bsp. 2, Abb. 2) und *B. cinerea* (Abb. 3).

Die mit Hilfe geeigneter Verfahren zur Identifizierung von Inhibitoren des 26S/20S Proteasoms identifizierten Verbindungen wirken gegen verschiedenste Pilze, wie z.B. humanpathogene Pilze oder pflanzenpathogene Pilze. Dazu gehören z.B. die folgenden Pilze aus dem Bereich der pflanzenschädigenden Pilze, wobei die Aufzählung jedoch nicht abschließend ist:
Plasmodiophoromycetes, Oomycetes, Chytridiomycetes, Zygomycetes, Ascomycetes, Basidiomycetes und Deuteromycetes,
Pythium-Arten, wie beispielsweise *Pythium ultimum,* Phytophthora-Arten, wie beispielsweise *Phytophthora infestans,* Pseudoperonospora-Arten, wie beispielsweise *Pseudoperonospora humuli* oder *Pseudoperonospora cubensis,* Plasmopara-Arten, wie beispielsweise *Plasmopara viticola,* Bremia-Arten, wie beispielsweise *Bremia lactucae,* Peronospora-Arten, wie beispielsweise *Peronospora pisi* oder *P. brassicae,* Erysiphe-Arten, wie beispielsweise *Erysiphe graminis,* Sphaerotheca-Arten, wie beispielsweise *Sphaerotheca fuliginea,* Podosphaera-Arten, wie beispielsweise *Podosphaera leucotricha,* Venturia-Arten, wie beispielsweise *Venturia inaequalis,* Pyrenophora-Arten, wie beispielsweise *Pyrenophora teres* oder *P. graminea* (Konidienform: Drechslera, Syn: Helminthosporium), Cochliobolus-Arten, wie beispielsweise *Cochliobolus sativus* (Konidienform: Drechslera, Syn: Helminthosporium), Uromyces-Arten, wie beispielsweise *Uromyces appendiculatus,* Puccinia-Arten, wie beispielsweise *Puccinia recondita,* Sclerotinia-Arten, wie beispielsweise *Sclerotinia sclerotiorum,* Tilletia-Arten, wie beispielsweise *Tilletia caries;* Ustilago-Arten, wie beispielsweise *Ustilago nuda* oder *Ustilago avenae,* Pellicularia-Arten, wie beispielsweise *Pellicularia sasakii,* Pyricularia-Arten, wie beispielsweise *Pyricularia oryzae,* Fusarium-Arten, wie beispielsweise *Fusarium culmorum,* Botrytis-Arten, Septoria-Arten, wie beispielsweise *Septoria nodorum,* Leptosphaeria-Arten, wie beispielsweise *Leptosphaeria nodorum,* Cercospora-Arten, wie beispielsweise *Cercospora canescens,* Alternaria-Arten, wie beispielsweise *Alternaria brassicae* oder Pseudocercosporella-Arten, wie beispielsweise *Pseudocercosporella herpotrichoides.*

Von besonderem Interesse sind z.B. auch *B. cinerea, Magnaporthe grisea, Cochliobulus heterostrophus, Nectria hematococcus* und Phytophtora Arten, Plasmodiophoromycetes, Oomycetes, Chytridiomycetes, Zygomycetes, Ascomycetes, Basidiomycetes und Deuteromycetes.

Die Inhibitoren des pilzlichen 20S Proteasoms können auch gegen die im Folgenden beispielhaft und nicht abschließend genannten human- oder tierpathogenen Pilze eingesetzt werden:
Dermatophyten, wie z.B. Trichophyton spec., Microsporum spec., *Epidermophyton floccosum* oder *Keratomyces ajelloi,* die z.B. Fußmykosen (Tinea pedis) hervorrufen,
Hefen, wie z.B. *Candida albicans,* die Soor-Ösophagitis und Dermatitis hervorruft, *Candida glabrata, Candida krusei* oder *Cryptococcus neoformans,* die z.B. pulmonale Cryptococcose und auch Torulose hervorrufen können,
Schimmelpilze, wie z.B. *Aspergillus fumigatus, A. flavus, A. niger,* die z.B. bronchopulmonale Aspergillose oder Pilzsepsis hervorrufen, Mucor spec., Absidia spec., oder Rhizopus spec., die z.B. Zygomykosen (intravasale Mykosen) hervorrufen, *Rhinosporidium seeberi,* der z.B. chronische granulomatöse Pharyngitis und Tracheitis hervorruft, *Madurella myzetomatis,* der z.B. subkutane Myzetome hervorruft, *Histoplasma capsulatum,* der z.B. retikuloendotheliale Zytomykose und M. Darling hervorruft, *Coccidioides immitis,* der z. B. pulmonale Coccidioidomykose und Sepsis hervorruft, *Paracoccidioides brasiliensis,* der z.B. brasilianische Blastomykose hervorruft, *Blastomyces dermatitidis,* der z.B. Gilchrist-Krankheit und nordamerikanische Blastomykose hervorruft, *Loboa loboi,* der z.B. Keloid-Blastomykose und Lobo's Krankheit hervorruft, und *Sporothrix schenckii,* der z.B. Sporotrichose (granulomatöse Hautmykose) hervorruft.

Fungizide Wirkstoffe, die mit Hilfe des pilzlichen 20S Proteasoms aus z.B. *S. cerevisiae* oder *U. maydis* gefunden werden, können also auch mit anderen zahlreichen Pilzspezies interagieren, wobei die Interaktion mit den unterschiedlichen in diesen Pilzen vorkommenden 26S bzw. 20S Proteasomen nicht immer gleich stark sein muss. Dies erklärt unter anderem die beobachtete Selektivität der an diesem Polypeptidkomplex wirksamen Substanzen.

Es sind bereits eine Reihe von Aktivitätstest für das Proteasom bekannt geworden und zum Teil auch käuflich zu erwerben (z.B. "20S Proteasome Assay Kit, SDS-Activated" von Calbiochem), die zur Prüfung der Aktivität des Proteasoms in kleinen Mengen und mit relativ großem zeitlichen Aufwand, d.h. mit mehreren Pipettierschritten, herangezogen werden können. Diese bekannten Aktivitätstest können z.B. genutzt werden, um die erfindungsgemäßen Fungizide zu identifizieren. Ein HTS-fähigen Verfahren, das die Identifizierung von Inhibitoren des 26S und/oder des 20S Proteasoms erlaubt, ist bislang jedoch noch nicht bekannt. Bekannte Verfahren, die im kleinen Maßstab zum Testen einzelner Verbindungen bzw. deren inhibitorischer Wirkung dienen können, sind aufgrund ihrer Komplexität ungeeignet, um ganze Substanzbibliotheken systematisch und mit ausreichender Verlässlichkeit in einem HTS- oder UHTS-System zu prüfen. Diese Systeme sind jedoch gegenüber anderen Verfahren besonders reizvoll, da eine sehr große Anzahl potentieller Inhibitoren in kurzer Zeit und guter Reproduzierbarkeit geprüft werden kann.

Dies ist insbesondere für das 20S Proteasom von Interesse, da hier mehrere enzymatische Aktivitäten in einem Enzym vereinigt sind, deren Hemmung durch einen potentiellen Inhibitor auch jeweils spezifisch beobachtet werden kann. Der damit verbundene Zeitaufwand kann durch die Nutzung eines HTS- oder UHTS-Systems deutlich verringert und die Wahrscheinlichkeit, einen Inhibitor mit wünschenswerten Eigenschaften zu identifizieren, deutlich erhöht werden. Diese unterschiedlichen enzymatischen Aktivitäten lassen sich anhand von für diese einzelnen Aktivitäten spezifische Substrate analysieren und voneinander trennen (McCormack et al. (1998), *Biochemistry 37,* 7792-7800), was insbesondere von Bedeutung ist, um die Spezifität und Wirkung der Inhibtoren zu beeinflussen und damit auch Inhibitoren bzw. Fungizide zu finden, die für Pilze bzw. bestimmte Gattungen oder Arten von Pilzen, nicht aber für andere Organismen wie z.B. Pflanzen, Insekten oder Säuger schädlich sind. Es sind bereits im humanen Bereich (humanes Proteasom) verschiedene spezifische Inhibitoren bekannt geworden. Es gibt jedoch auch Inhibitoren, die mehrere Aktivitäten des Proteasoms beeinflussen.

Aufgabe der vorliegenden Erfindung war es deshalb auch, ein für HTS- sowie UHTS-Verfahren zugängliches *in vitro* Verfahren zur Verfügung zu stellen, das die Identifizierung von gegebenenfalls spezifischen Inhibitoren des Proteasoms im großen Maßstab ermöglicht, und gegebenenfalls eine nachträgliche Zuordnung zu bestimmten enzymatischen Aktivitäten des Polypeptidkomplexes erlaubt. Durch die Hemmung geeigneter proteolytischer Aktivitäten kann so z.B. die Toxizität für höhere Eukaryonten reduziert werden. Darüber hinaus können die einzelnen biologischen Aktivitäten getrennt und entsprechend spezifisch moduliert werden. In der Medizin ist dies wichtig, um z.B. zytotoxische Effekte zu reduzieren, indem die Aktivitäten, die nicht Ziel der Therapie sind, nicht inhibiert werden.

Es wurde nun ein Verfahren gefunden, in dem anhand von HTS-fähigen *in vitro* Tests ermöglicht wird, Inhibitoren des pilzlichen Proteasoms aus einer Vielzahl zu testender Verbindungen, so genannte Kandidatenverbindungen, herauszufiltern und gegebenenfalls einer spezifischen proteolytischen Aktivität des 20S Proteasoms zuzuordnen.

Das erfindungsgemäße Verfahren beruht dabei auf zwei Verbesserungen des Gesamtverfahrens. Zum einen wird mit der vorliegenden Erfindung eine effiziente, zugleich aber bedeutend schnellere und einfachere Herstellung von Proteasomen-Extrakten ermöglicht, und zum zweiten wurde eine Erhöhung der Stabilität der Testkomponenten erzielt, wodurch auch eine Reduzierung der für die Durchführung eines Aktivitätstest nötigen Schritte erreicht wurde. Die schnelle und effiziente Reinigung der Proteasomen beruht dabei auf der Einführung eines Inkubationsschrittes bei erhöhter Temperatur, der nachfolgend näher beschrieben wird. Die Erhöhung der Stabilität der Reaktionskomponenten bei Erreichen eines hervorragenden Signal-Hintergrund-Verhältnisses und gleichzeitiger Reduktion der nötigen Schritte beim Aktivitätstest wurde überraschenderweise durch eine Erhöhung der DMSO-Konzentration auf ungewöhnlich hohe Werte ermöglicht. Dabei wurde ein Optimum für die nötige DMSO-Konzentration ermittelt.

Das vorstehend genannte Verfahren zur Herstellung eines aktiven 20S-Proteasomen-Extraktes wurde bislang nicht beschrieben. Die genannten HTS-fähigen Verfahren zum Identifizieren von Inhibtoren von 26S/20S Proteasomen, insbesondere von Proteasomen aus Pilzen, wurden bislang ebenfalls nicht beschrieben.

Gegenstand der vorliegenden Erfindung ist ein Verfahren zum Identifizieren von Fungiziden durch Testen einer Kandidatenverbindung in einem 20S-Proteasomen-Hemmtest. Besonders bevorzugt wird das Verfahren zur Identifizierung von Inhibitoren des pilzlichen 20S Proteasoms, d.h. zur Identifizierung von Fungiziden verwendet.

Neben der Inhibition des Proteasoms *in vitro* ist es - um die betreffenden Inhibitoren auch als Fungizide verwenden zu können - entscheidend, dass die identifizierten Verbindungen auch *in vivo* wirksam sind, also tatsächlich als Fungizide, z.B. in der Landwirtschaft, verwendet werden können. Viele Inhibitoren, die in *in vitro* Tests gefunden werden, sind aus verschiedenen Gründen nicht in der Lage, den Zielorganismus selbst zu schädigen. So wurde wie vorstehend beschrieben im Stand der Technik keine Wirkung von bekannten Inhibitoren des humanen Proteasoms bei Pilzen gefunden.

Im Rahmen der vorliegenden Erfindung wird gezeigt, dass die z.B. anhand des nachstehend beschriebenen, erfindungsgemäßen Verfahrens identifizierten Inhibitoren des Proteasoms, jedoch auch bereits bekannte Inhibitoren z.B. des menschlichen Proteasoms, wider erwarten wirksame Fungizide sind, also Substanzen, die nicht nur *in vitro* eine inhibierende Wirkung zeigen, sondern auch *in vivo* verwendet werden können, um die Funktion des pilzlichen Proteasoms zu stören und den betreffenden Pilz zu schädigen oder abzutöten.

Zur Identifizierung der Inhibitoren des pilzlichen 20S Proteasoms muss dabei nicht das nachstehend beschriebene, erfindungsgemäße Verfahren verwendet werden. Vielmehr können alle bekannten Verfahren zur Bestimmung der Aktivität des 20S Proteasoms herangezogen werden, insofern sie die Beurteilung der inhibitorischen Wirkung einer Kandidatenverbindung auf das pilzliche 20S Proteasom zulassen.

Gegenstand der vorliegenden Erfindung ist deshalb auch ein Verfahren zum Identifizieren von Fungiziden durch Inkontaktbringen von 20S Proteasomen, bevorzugt von 20S Proteasomen aus Pilzen, mit einer Kandidatenverbindung bei Anwesenheit eines Substrats und bei Anwesenheit von 2 bis 10% (v/v) Dimethylsulfoxid und anschließende Auswahl der Verbindung(en), die die enzymatische Aktivität des 20S Proteasoms inhibieren. Vorzugsweise erfolgt das Verfahren so, dass die Messung der enzymatischen Aktivität des 20S Proteasoms bei An- und bei Abwesenheit eines Kandidatenmoleküls erfolgt, wobei solche Kandidatenverbindungen ausgewählt werden, die die enzymatische Aktivität des Proteasoms im Vergleich zur Kontrolle bei Abwesenheit einer Kandidatenverbindung reduzieren, also Proteasomen-Inhibitoren sind.

Der Begriff "Proteasomen-Inhibitor", wie er hierin gebraucht wird, bezeichnet eine Substanz, die direkt oder indirekt zumindest eine, gegebenenfalls aber auch mehrere der vorstehend genannten enzymatischen Aktivitäten inhibiert. Ein solcher Inhibitor ist vorzugsweise spezifisch, d.h. er inhibiert die Proteasomen-Aktivität bei einer Konzentration die niedriger ist als die Konzentration eines Inhibitors, die benötigt wird, um einen anderen, damit nicht verbundenen Effekt hervorzurufen. Vorzugsweise ist die Konzentration zweifach niedriger, insbesondere bevorzugt fünffach niedriger und ganz besonders bevorzugt zumindest zehnfach oder 20fach niedriger als die Konzentration einer Verbindung, die zum Hervorrufen eines unspezifischen Effekts benötigt wird.

Der Begriff "Proteasomen-Aktivität", wie er hierin verwendet wird, bezieht sich auf eine oder mehrere der vorstehend genannten enzymatischen Aktivitäten des 20S bzw. 26S Proteasoms.

Im Rahmen der vorliegenden Erfindung wurden beispielhaft jeweils 20S Proteasomen aus *S. cerevisiae, U. maydis und B. cinerea* verwendet, um Inhibitoren des 26S und/oder 20S Proteasoms zu identifizieren. Es ist jedoch ebenso möglich, anstelle des 20S Proteasoms aus *S. cerevisiae, U. maydis oder B. cinerea* 20S Proteasomen aus anderen Pilzen bzw. aus anderen, nicht pilzlichen Organismen zu verwenden, um Inhibitoren zu identifizieren. Dies wird daran deutlich, dass auch Inhibitoren des humanen Proteasoms als Fungizide wirken können.

Der Aufschluss der Zellen kann durch verschiedene, dem Fachmann bekannte Techniken erfolgen. Die anschließende Reinigung/Isolierung der Proteasomen kann ebenfalls mit bekannten Verfahren (z.B. Groll et al. (1997), Structure of the 20S Proteasom from yeast at 2.4 Å resolution, *Nature 386,* 463-471; EP 345 750 A2; WO 98/42829; JP 05292964 A; JP 06022759 A) erzielt werden. Die Ausdrücke "Isolierung", "Anreicherung" oder "Reinigung", wie sie hierin verwendet werden, bedeuten, dass die 20S Proteasomen von anderen Proteinen oder anderen Makromolekülen der Zelle oder des Gewebes soweit abgetrennt werden, dass eine spezifische Messung ihrer enzymatischen Aktivität bzw. deren Inhibition erfolgen kann. Vorzugsweise ist eine die 20S Proteasomen enthaltende Zusammensetzung hinsichtlich des Proteasomengehalts gegenüber einer Präparation aus den Wirtszellen mindestens 10-fach und besonders bevorzugt mindestens 100-fach angereichert. Die bekannten Verfahren sind relativ komplex und umfassen eine Reihe von Schritten (vgl. z.B. WO 98/42829). Gerade für die Verwendung von Proteasomen in erfindungsgemäßen Verfahren ist es erstrebenswert, in kurzer Zeit 20S Proteasomen zu gewinnen, die zumindest ebenso aktiv sind, wie die mit aufwendigen Methoden des Standes der Technik hergestellten 20S Proteasomen, und die eine Messung ihrer enzymatischen Aktivität und deren Inhibition zulassen.

Im vorliegenden erfindungsgemäßen Verfahren werden die Hefezellen nach dem Fachmann bekannten Verfahren aufgeschlossen, vorzugsweise mechanisch (z.B. durch Verwendung einer French Press oder eines Hochdruckhomogenisators) im später verwendeten Testpuffer (z.B. 50 mM Tris-HCI, pH 7,5, 10 mM EDTA). Geeignet für das erfindungsgemäße Verfahren sind z.B. auch käufliche Hefen (Bäckerhefe), wie sie zum Backen verwendet werden. Die Proteasomen befinden sich dann in der löslichen Fraktion und können durch Zentrifugation von unlöslichen Bestandteilen und Zellresten getrennt werden, wobei dieser Schritt gemäß dem erfindungsgemäßen Verfahren jedoch nicht unbedingt durchgeführt werden muss. Die Suspension bzw. die lösliche Fraktion wird dann für ca. 20 bis 75 Minuten bei ca. 50°C bis 70°C, bevorzugt bei 55°C bis 65°C inkubiert. Besonders geeignet ist eine Inkubation bei ca. 60°C für etwa 60 Minuten. Dabei wird die Erkenntnis genutzt, dass das Protein im Gegensatz zu den anderen noch anwesenden Proteinen bei dieser Temperatur stabil ist. Bei diesem erfindungsgemäßen Reinigungsschritt fallen deshalb temperaturlabile Proteine aus und können vom temperaturstabilen 20S Komplex durch Zentrifugation abgetrennt werden. Die Inkubation kann demnach abgebrochen werden, wenn keine Zellbestandteile mehr denaturiert werden und ausfallen. Die Verwendung eines Hitzeschritts zur Trennung des Proteasoms von anderen Bestandteilen der Zelle wird im Stand der Technik nicht beschrieben. Er ermöglicht jedoch eine schnelle, einfache und sehr effiziente Reinigung von Proteasomen in nur einem Schritt, wobei die so gereinigten Proteasomen nach der Entfernung der ausgefallenen Zellbestandteile direkt in Aktivitäts- bzw. Hemmtests verwendet werden können. Zum Überstand kann dann z.B. Natriumazid zugegeben werden, um das Wachstum von Mikroorganismen zu verhindern. Durch das erfindungsgemäße Verfahren wird außerdem sichergestellt, dass bevorzugt 20S Proteasomen erhalten werden, nicht 26S Proteasomen, die für die Identifizierung von Inhibitoren eher ungeeignet sind, weil proteolytisch aktive Bereiche in vollständigen 26S Proteasomen nicht zugänglich sein können. Aus 30 g Hefe (Feuchtgewicht) lassen sich auf diese einfache Weise ausreichend Proteasomen für eine Verwendung in einem HTS-fähigen Verfahren zur Identifizierung von Inhibitoren gewinnen.

Bevorzugt werden die in das erfindungsgemäße Verfahren eingesetzten 20S Proteasomen durch ein Verfahren aus eukaryontischen Zellen, vorzugsweise aus Pilzen aufgereinigt, welches dadurch gekennzeichnet ist, dass man
a) Zellen, vorzugsweise Pilzzellen, mit üblichen Verfahren aufschließt,
b) die entstandene Suspension auf 50°C bis 70°C erhitzt bis die temperatursensitiven Bestandteile ausgefallen sind, und
c) die Proteasomen durch Abzentrifugieren der unlöslichen Bestandteile gewinnt.

Das Verfahren zum Aufreinigen von 20S Proteasomen kann bei verschiedenen Organismen verwendet werden. So wird im Rahmen der vorliegenden Erfindung demonstriert, dass mit Hilfe des erfindungsgemäßen Verfahrens z.B. aus *S. cerevisiae, U. maydis* und *B. cinerea* die 20S Proteasomen mit hoher Aktivität gewonnen werden können. In gleicher Weise kann das Verfahren auch bei anderen eukaryontischen, insbesondere bei pilzlichen Zellen genutzt werden.

Verfahren zum Testen der enzymatischen Aktivität von 20S Proteasomen und zum Testen der Hemmung bzw. Inhibition dieser Aktivität sind aus der Literatur bekannt. Diese Testsysteme machen sich z.B. bestimmte Substrate des Proteasoms zu nutze, deren enzymatischer Abbau zur Freisetzung einer fluorometrisch oder colorimetrisch bestimmbaren Gruppe führt. Die verwendeten Verfahren beruhen dabei auf der schrittweisen Zugabe der einzelnen Komponenten (z.B. Reaktionspuffer, Aktivierungspuffer, 20S Proteasomen, Substratlösung, Inhibitor) und einer anschließenden Messung der sich ändernden Fluoreszenz oder Absorption (siehe z.B. "20S Proteasome Assay Kit, SDS-Activated von Calbiochem" Cat. Nr. 539158). Dabei muss darauf Rücksicht genommen werden, dass die Stabilität des Substrats unter Versuchsbedingungen gering ist. Die Peptidsubstrate, wie z.B. Suc-LLVY-AMC, sind aufgrund ihrer geringen Löslichkeit und Stabilität relativ instabil und deshalb wenig geeignet für Messungen mit hoher Zeitdauer von mehreren Stunden, wie es z.B. in HTS-/UHTS-Systemen bei Messung einer extrem großen Anzahl von Proben, die vorgelegt und gemessen werden müssen, unumgänglich ist. Die Substrate bzw. die darauf basierenden Aktivitätstests eigenen sich deshalb bislang nur für Einzelexperimente mit geringem Zeitaufwand, die gewährleisten, dass das verwendete Peptidsubstrat noch intakt ist. So wird das Substrat Suc-LLVY-AMC als gering löslich (50 mg/ml in DMSO) und als lichtempfindlich beschrieben. Ein typischer Versuchsansatz ("20S Proteasome Assay Kit, SDS-Activated" von Calbiochem) beruht auf der Durchführung der folgenden grundsätzlichen Schritte: Ein Reaktionspuffer wird vorgelegt, in einem zweiten Schritt Aktivierungspuffer (enthaltend z.B. SDS) zupipettiert und beide Komponenten gemischt. Zu dieser Lösung werden in einem dritten Schritt 20S Proteasomen zugefügt. In einem vierten Schritt wird das Substrat zugefügt, wodurch die Reaktion gestartet wird. In einem fünften Schritt wird der Inhibitor zugefügt, wobei die letzten beiden Schritte wahlweise auch in umgekehrter Reihenfolge erfolgen können.

Um einen solchen Aktivitätstest für HTS-/UHTS-Verfahren verwendbar zu machen, müsste die große Zahl an Pipettierschritten verringert werden. Dazu wiederum müssten Mischungen der hier einzeln pipettierten Komponenten über einen längeren Zeitraum stabil bleiben, um dem in der Regel hohen Zeitbedarf für die Vorbereitung der Proben und das Messen zahlreicher Kandidatenverbindungen Rechnung zu tragen. Die enzymatische Aktivität des Proteasoms darf dabei ebenso wenig wie die des Substrats beeinträchtigt werden, um das benötigte Signal-Hintergrund-Verhältnis zu erhalten. Die Aufgabe der vorliegenden Erfindung war es deshalb, die bekannten Testsysteme so weiterzuentwickeln, das deren Verwendung in HTS-/UHTS-Verfahren ermöglicht wird.

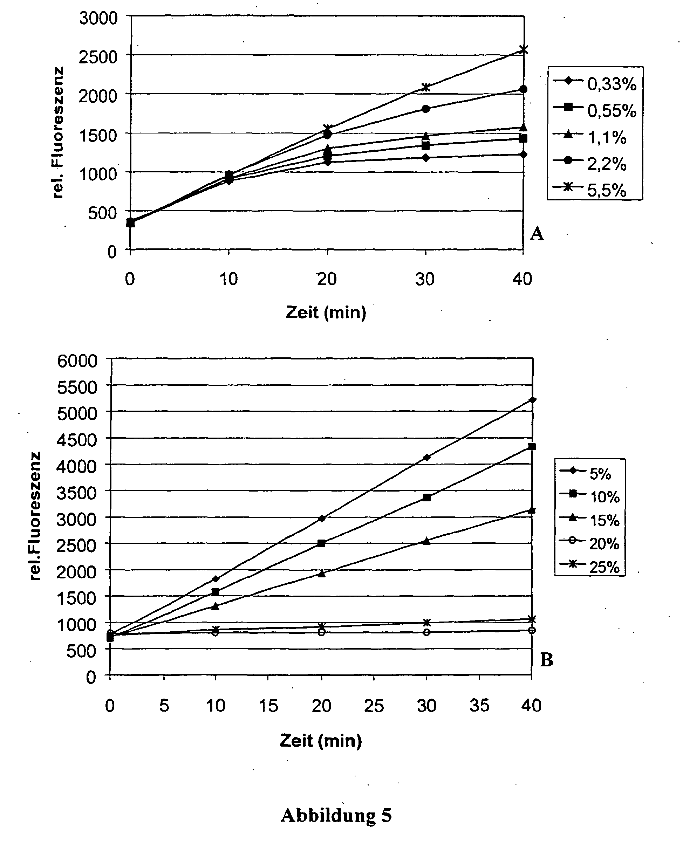

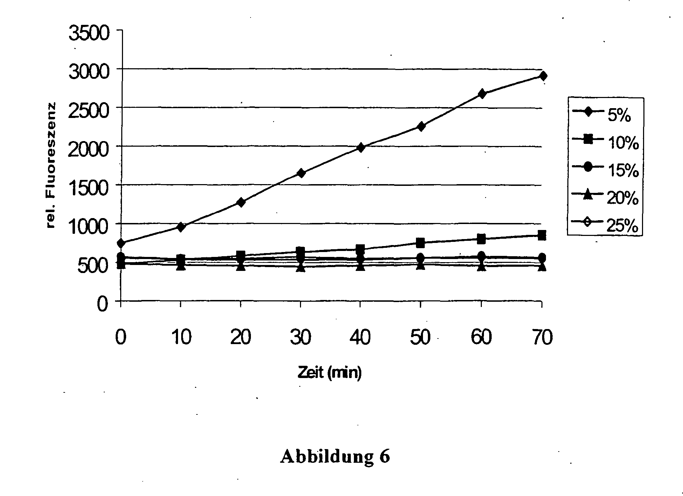

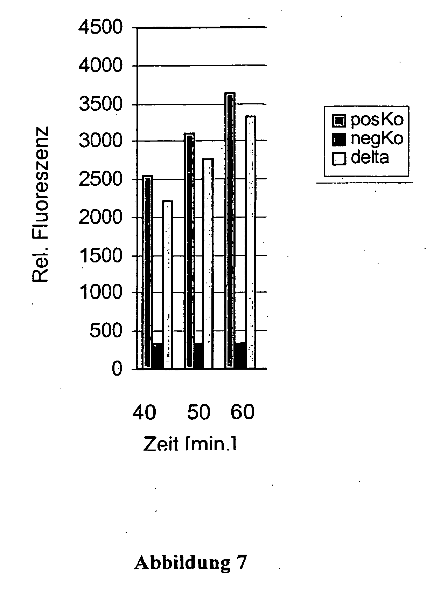

Die Lösung des Problems beruht darauf, im Reaktionsansatz DMSO (Dimethylsulf oxid) zu verwenden bzw. die DMSO-Konzentration im Reaktionsansatz zu erhöhen. Es wurde gefunden, dass z.B. die chymotryptische Aktivität des Proteasoms bei höheren DMSO-Konzentrationen abnimmt (Abb. 5(B)), während die Stabilität des Substrats bei höheren DMSO-Konzentrationen zunimmt. Ein Optimum für die Substratstabilität bei optimalem Erhalt der chymotryptischen Aktivität ergab sich schließlich für eine DMSO-Konzentration von 2 bis 10% DMSO (v/v), wobei eine Konzentration von 3 bis 7% besonders erstrebenswert ist. Insbesondere geeignet ist eine DMSO-Konzentration von 4,5 bis 6% (Abb. 5(A) und (B)). Bei einer Konzentration von 5% DMSO bleibt die Proteasomen-Aktivität sogar über 72 h, und nicht nur für ca. 1 h wie im Stand der Technik beschrieben, stabil (Abbildung 6).

Damit wird im erfindungsgemäßen Verfahren die Stabilität des Substrats deutlich erhöht, ohne die Aktivität des Proteasoms zu beeinträchtigen. Neben einer deutlich erhöhten Zeitspanne, die für die Durchführung des Aktivitäts- bzw. Hemmtests genutzt werden kann, können gemäß der vorliegenden Erfindung die benötigten Lösungen auch bereits vorab zusammengestellt und über einen längeren Zeitraum gelagert werden (Abbildung 6), und damit die Anzahl der nötigen Pipettierschritte auf drei Pipettierschritte anstelle von bis zu fünf Schritten in den bekannten Verfahren (siehe oben) reduziert werden. Dabei wird z.B. die Kandidatenverbindung in einem geeigneten Puffer vorgelegt, und in einem zweiten Schritt die Substratlösung enthaltend eine geeignete Menge von DMSO zugegeben, die dann auch bereits den Aktivator SDS enthalten kann. In einem dritten Schritt wird die 20S Proteasomen-Lösung zugegeben und die Reaktion damit gestartet. Die Reihenfolge der Zugabe der einzelnen Komponenten kann dabei auch variiert werden.

Die im erfindungsgemäßen Verfahren verwendeten Temperaturen können sehr variabel gestaltet werden. Es können Temperaturen von 15°C bis 80°C verwendet werden, insbesondere von 25°C bis 50°C oder einfach Raumtemperatur. Besonders bevorzugt wird eine Temperatur von 37°C verwendet.

Im erfindungsgemäßen Verfahren können auch Aktivatoren des 20S Proteasoms eingesetzt werden, die im Reaktionsansatz enthalten sind. Bevorzugte Aktivatoren sind die in Coux et al. (1995), *Ann. Rev. Biochem. 65,* 801-847, beschriebenen Aktivatoren, wobei die Aktivatoren SDS (sodium dodecyl sulfate) und PA28α (Knowlton et al. (1997), *Nature 390,* 639-643), insbesondere hervorgehoben werden.

Die Inhibition der enzymatischen Aktivität des 20S Proteasoms durch die Kandidatenverbindung kann durch einen Vergleich der Fluoreszenz oder der Absorption bei An- und bei Abwesenheit der Kandidatenverbindung verfolgt werden.

Gegenstand der vorliegenden Erfindung ist auch ein Verfahren zum Identifizieren von Fungiziden wie vorstehend und nachstehend beschrieben, an das sich ein *in vivo* Test zur Bestimmung der fungiziden Aktivität anschließen kann. Vorzugsweise umfasst dieser Test das Inkontaktbringen des gefundenen Inhibitors mit zumindest einer Pilzspezies und die anschließende Prüfung der Schädigung des Pilzes.

Der Begriff "20S Proteasomen-Hemmtest" wie er hierin verwendet wird, bezieht sich dabei insbesondere auf die Messung der enzymatischen Aktivität von 20S Proteasomen anhand der aus der Freisetzung einer fluorogenen oder colorimetrisch bestimmbaren Gruppe aus einem Substrat des 20S Proteasoms resultierenden Fluoreszenz oder Absorption, bzw. der Inhibition dieser Freisetzung durch einen Inhibitor der betreffenden Aktivität. Insbesondere ist das erfindungsgemäße Verfahren anzuwenden, wenn der Hemmtest auf der Verwendung eines labilen, durch DMSO stabilisierbaren Substrats beruht.

Im natürlichen Kontext hydrolysiert das 20S Proteasom ungeschützte Peptide, so dass auch ungeschützte Substrate im erfindungsgemäßen Verfahren verwendet werden können. Zu berücksichtigen ist jedoch, dass die Stabilität gegenüber geschützten Substraten weiter reduziert sein kann. Neben dem Fluorophor AMC können auch andere Fluorophore oder auch Chromophore verwendet werden, so z.B. die Fluorophore 2-Naphthylamid (2NA), 4-Methoxy-2-naphthylamid oder 6-Aminochinolin. Colorimetrisch bestimmbare Gruppen sind z.B. p-Nitroanilid (pNA), p-Phenylazoanilid oder 3,5-Dibromo-4-hydroxy-anilid. Für die verschiedenen enzymatischen Aktivitäten des Proteasoms sind unter anderem die nachstehend genannten Peptidsubstrate bekannt, deren Abbau fluorimetrisch oder colorimetrisch verfolgt werden kann. Solche Substrate sind zum Beispiel Z-Leu-Leu-Leu-AMC (Z-LLL-AMC) zur Messung der chymotryptischen Aktivität, Z-Leu-Leu-Glu-AMC (Z-LLE-AMC) zur Messung der Peptidylglutamylpeptid-hydrolisierenden Aktivität, Z-Val-Lys-Met-AMC (Z-VKM-AMC) zur Messung der chymotryptischen Aktivität, Suc-Leu-Tyr-AMC (Suc-LY-AMC) zur Messung der chymotryptischen Aktivität, Z-Leu-Leu-Glu-2NA (*N*-(*N*-Carbobenzyloxycarbonyl-leucyl-leucyl-arginyl)-2-naphthylamin) oder Suc-Leu-Leu-Val-Tyr-AMC (Suc-LLVY-AMC) zur Messung der PGPH bzw. der chymotrypsinartigen Aktivität, die alle käuflich zu erwerben sind (siehe auch WO 00/23614). Als Substrate können auch die vorstehend genannten Substrate, insbesondere das Suc-LLVY-AMC verwendet werden, die andere Schutzgruppen tragen. Häufig verwendete Schutzgruppen sind z.B. Benzyloxycarbonyl (auch "Z" genannt, s.o.) oder N-tert-Butoxycarbonyl (t-boc).

Für das erfindungsgemäße Verfahren kann zumindest ein Substrat ausgewählt aus der vorstehend genannten Gruppe von Substraten verwendet werden, wobei ein Substrat ausgewählt aus der folgenden Gruppe von Substraten bevorzugt verwendet wird: Z-LLL-AMC, Z-LLE-AMC, Z-VKM-AMC, Suc-LY-AMC, und insbesondere Suc-LLVY-AMC.

Darüber hinaus sind kovalente und kompetetive Inhibitoren bekannt, so z.B. *clasto-*Lactacystin β-Lacton, Epoxomicin (inhibiert die chymotrypsinartige, trypsinartige und Peptidylglutamylpeptid-hydrolisierende Aktivität des Proteasoms), Z-Leu-Leu-Leu-B(OH)₂, Z-Leu-Leu-Leu-CHO (MG-132, inhibiert die Degradation über den Ubiquitin-Weg), Z-Leu-Leu-Nva-CHO (MG-115, Carbobenzoxy-L-leucyl-L-leucyl-L-norvalinal, inhibiert spezifisch die chymotrypsinartige Aktivität des Proteasoms), Z-lleu-Glu(OtBu)Ala-Leu-CHO, Z-Leu-Leu-Phe-CHO (inhibiert die chymotrypsinartige Aktivität), Ac-Leu-Leu-Nle-CHO (MG-101, Ac-Leu-Leu-Norleucinal, inhibiert die chymotrypsinartige und die Peptidaseaktivität) oder Ac-Leu-Leu-Met-CHO (inhibiert die Peptidaseaktivität), die ebenfalls alle käuflich zu erwerben sind (siehe auch Mellgren (1997): Specificities of Cell Permeant Peptidyl Inhibitors for Proteinase Activities of µ-Calpain and the 20S Proteasome. *J. Biol. Chem. 272,* 29899-29903; Bogyo et al. (1998): Substrate binding and sequence preference of the proteasome revealed by active-site-directed affinity probes. *Chem. Biol. 5,* 307-320). Solche bekannte Inhibitoren können so auch zum Überprüfen des erfindungsgemäßen Verfahrens herangezogen werden (siehe Bsp. 3). In weiteren Tests an Pilzzellen wurde festgestellt, dass diese bekannten Inhibitoren überraschenderweise auch eine fungizide Wirkung aufweisen und deshalb auch als Fungizide oder zur Herstellung eines Medikaments zur Behandlung von Pilzbefall verwendet werden können.

Die jeweilige enzymatische Aktivität oder auch mehrere Aktivitäten des Proteasoms können grundsätzlich auf Basis der vorstehend genannten, bekannten Substrate bzw. deren Spaltprodukte fluorimetrisch oder colorimetrisch gemessen, und die Inhibtion dieser Aktivität mit Hilfe eines der vorstehend genannten, bekannten Inhibitoren geprüft werden. Dazu können je nach Zielaktivität die passenden Substrate und gegebenenfalls Inhibitoren gewählt werden. Die mit einem erfindungsgemäßen Verfahren bzw. einem spezifischen Substrat gefundenen Inhibitoren können dann direkt als Inhibitoren einer oder mehrerer spezifischer Aktivitäten erkannt werden.

Die Wahl des Substrats muss jedoch die Auswertung der Messergebnisse ermöglichen, d.h. eine Inhibition muss deutlich erkennbar sein. Als statistische Parameter kann z.B. das Signal-Hintergrund Verhältnis herangezogen werden. Eine geeignete Größe zur Bestimmung der Qualität eines Screeningverfahrens bzw. eines Hemmtests ist auch der z-Faktor. In den z-Faktor gehen neben der Differenz aus Signal und Hintergrund auch die Streuung aller Messwerte in die Berechnung mit ein. Der z-Faktor berechnet sich wie folgt: z-Faktor = 1-((3 x Standardabweichung posKo + 3 x Standardabweichung negKo) / (Mittelwert posKo - Mittelwert negKo)), wobei "posKo" für die Positivkontrolle steht und "negKo" für die Negativkontrolle (Zhang et al. (1999): A Simple Statistical Parameter for Use in Evaluation and Validation of High Throughput Screening Assays. *J. Biomol. Screen. 4(2)*:67-73).

Ein z-Faktor größer 0,7 gilt als exzellent, ein z-Faktor von 0,15 bis 0,7 als gut, ein z-Faktor von 0 bis 0,15 als noch ausreichend und ein z-Faktor von kleiner 0 als nicht mehr auswertbar.

Der z-Faktor im erfindungsgemäßen Verfahren liegt nach ca. 30 Minuten über 0,7.

Im Folgenden wird beispielhaft eine mögliche Ausgestaltung eines erfindungsgemäßen Verfahrens gezeigt, wobei ein bekannter Inhibitor, MG-132, verwendet wird, um das Prinzip des Verfahrens zu demonstrieren.

Die zu prüfende enzymatische Aktivität des Proteasoms bzw. deren Inhibition kann wie im vorliegenden Beispiel durch den Anstieg der Fluoreszenz von freigesetztem AMC aus dem bekannten Peptidsubstrat Suc-LLVY-AMC (Suc-Leu-Leu-Val-Tyr-AMC; Stein et al. (1996) *Biochemistry 35,* 3899) wie nachstehend schematisch dargestellt nachgewiesen werden:

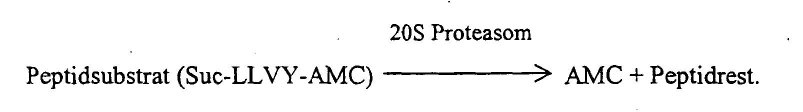

Suc-LLVY-AMC ist ein fluorogenes Substrat zur Messung der chymotrypsinartigen Aktivität des 20S Proteasoms. Das abgespaltene AMC (7-Amino-4-methoxy-coumarin) weist ein Anregungsmaximum bei einer Wellenlänge von 380 nm und ein Emissionsmaximum bei 460 nm auf. Der Kᵢ für den Inhibitor liegt bei 5 nM.

Grundsätzlich können jedoch neben dem hier beispielhaft genannten Substrat Suc-LLVY-AMC auch andere Substrate des Proteasoms verwendet werden, z.B. die vorstehend angegebenen bekannten Substrate.

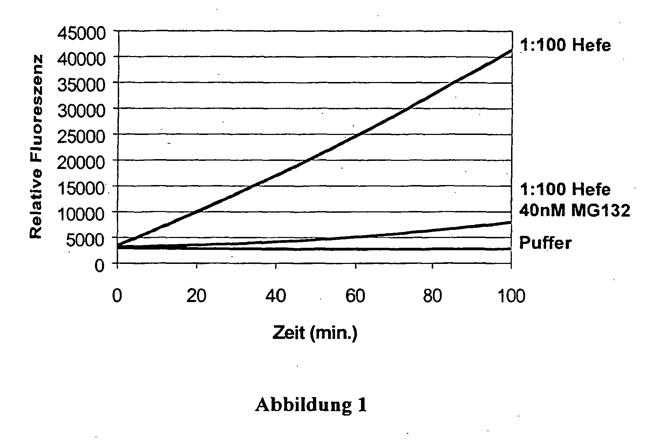

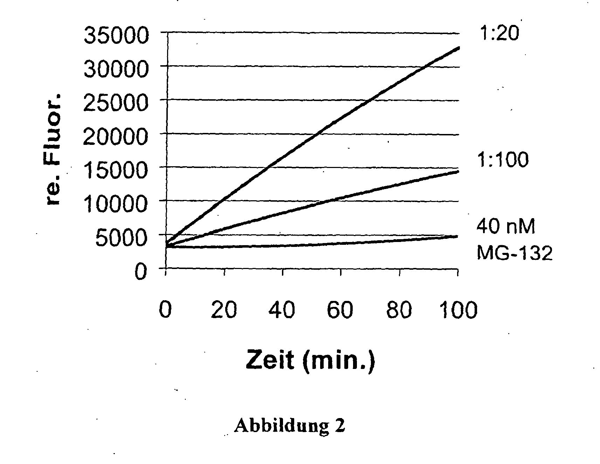

Die auf diese Weise messbare proteolytische Aktivität kann durch einen Inhibitor wie z.B. MG-132 beeinflusst werden, was sich an einer geringeren relativen Fluoreszenz bemerkbar macht (Abbildungen 1 und 2). MG-132 ist ein bekannter, reversibler Proteasomen-Inhibitor, der Zellwände durchdringen kann (Lee, D.H., Goldberg A. (1989) *J. Biol. Chem. 271*, 27280). Die Inhibition der Proteasomen-Aktivität durch den bekannten Inhibitor verringert die relative Fluoreszenz deutlich. Als Negativ-Kontrolle wurde die Reaktion ohne Proteasomen durchgeführt. Die gemäß dem erfindungsgemäßen Verfahren durchgeführte Reaktion gestattet problemlos die Erkennung einer Inhibition der Proteasomen-Aktivität und damit die Identifizierung von Inhibitoren des Proteasoms.

Es können auch andere Inhibitoren zum Testen des Verfahrens verwendet werden, so z.B. die vorstehend angegebenen bekannten Inhibitoren der Proteasomen-Aktivität.

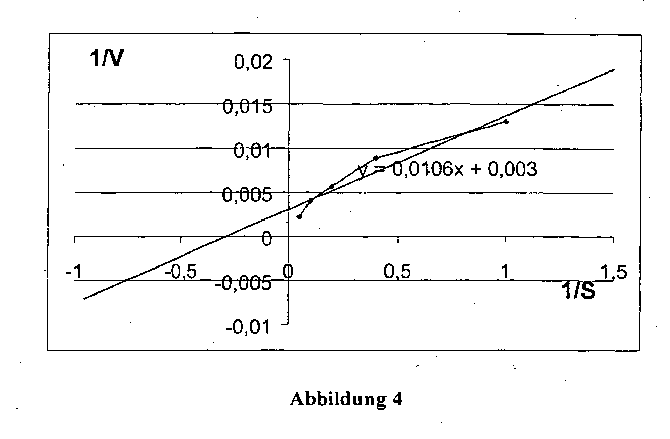

Um optimale Bedingungen für ein Verfahren zum Identifizieren von Inhibitoren von Proteasomen bzw. zur Bestimmung der Aktivität der Polypeptidkomplexe zu ermitteln, ist es vorteilhaft, den jeweiligen K_{M}-Wert des verwendeten Substrates zu bestimmen. Dieser gibt Aufschluss über die bevorzugt zu verwendende Konzentration des bzw. der Substrate. Für das beispielhaft verwendete Substrat Suc-LLVY-AMC konnte ein K_{M} von 5 µM bestimmt werden (siehe Abbildung 4).

Es versteht sich von selbst, dass neben dem besonders bevorzugten, vorstehend beschriebenen erfindungsgemäßen Verfahren zum Identifizieren von Inhibitoren pilzlicher 20S Proteasomen bzw. von Fungiziden auch alle herkömmlichen, dem Fachmann bekannten Verfahren verwendet werden können, die z.B. in WO 00/23614, Groll et al. (1997), *Nature 386,* 463, Mellgren (1997): *J. Biol. Chem. 272,* 29899, Meng et al. (1999), *PNAS 96,* 10403, McCormack et al. (1998), *Biochemistry 37*, 7792, Driscoll und Goldberg (1990), *J. Biol. Chem.* 265, 4789 oder Orlowski et al. (1993): *Biochemistry 32,* 1563 beschrieben sind, oder die käuflich zu erwerben sind (z.B. von Calbiochem). Dabei verwendete Substrate des Proteasoms sind neben den bereits vorstehend beschrieben z.B. auch Lysozym, alpha-Lactalbumin, beta-Lactoglobulin, β-Insulin oder Ornithin-Decarboxylase. Wenn die Aktivität des ganzen 26S Proteasoms gemessen werden soll, ist das Substrat vorzugsweise ubiqutiniert, oder der Reaktionsansatz enthält zusätzlich Ubiquitin und Ubiquitinierungs-Enzyme.

Mit Hilfe des vorstehend beispielhaft beschriebenen Verfahrens konnten Verbindungen identifiziert werden, die neben der *in vitro* Inhibition der 20S Proteasomen *in vivo* eine fungizide Wirkung zeigen.

In Tabelle I werden beispielhaft Verbindungen gezeigt, die im erfindungsgemäßen Verfahren eine inhibitorische Wirkung und in folgenden *in vivo* Tests, hier z.B. dem so genannten Agar-Diffusions-Test, eine fungizide Wirkung gegenüber verschiedenen Pilzen aufweisen. Dabei wurden beispielhaft die folgenden Pilze verwendet: *Botrytis cinerea* (BOTRCI), *Ustilago maydis* (USTIMA), *Pyricularia orycae* (PYRIOR), *Fusarium culmorum* (FUSACU), *Rhizoctonia solani* (RHIZSO), *Pseudo-cercosporella herpotrichoides* (PSDCHW). Der "MHK"-Wert beschreibt die "minimale Hemmkonzentration". Er gibt die kleinste Konzentration wieder (in ppm), bei der ein Wirkstoff das Wachstum des Pilzes unterbindet. Je kleiner der MHK-Wert also ist, desto größer ist die Wirksamkeit der getesteten Substanz.

**Tabelle I**

| **Beispiel** | **Verbindung** | **Organismus** | **MHK** |
|---|---|---|---|
| **1** | Carbobenzoxy-L-leucyl-L-leucyl-L- | BOTRCI | 58,6 |
| | norvalinal | FUSACU | >1000 |
| | (MG-115) | PSDCHR | >1000 |
| | | PYRIOR | 2,0 |
| | | RHIZSO | >1000 |
| | | USTIMA | 750 |
| **2** | Aprotinin | BOTRCI | 339,3 |
| | | FUSACU | >1000 |
| | | PSDCHR | >1000 |
| | | PYRIOR | 18,0 |
| | | RHIZSO | >1000 |
| | | USTIMA | 750 |

Damit kann gezeigt werden, dass die mit Hilfe eines erfindungsgemäßen Verfahrens identifizierten Inhibitoren von 26S/20S Proteasomen sowie bekannte Inhibitoren des humanen 26S/20S Proteasoms geeignet sind, Pilze zu schädigen oder zu töten.

Der Agar-Diffusions-Test ist ein einfacher Weg zur Bestimmung der Empfindlichkeit von Mikroorgansimen gegenüber antimikrobiellen Wirkstoffen (siehe z.B. Mitchell und Carter (2000): Modeling antimocrobial activity of Clorox^{™} using an agardiffusion test: a new twist on an old experiment. *Bioscene 26(3),* 9-13). Dabei wird eine mit dem Mikroorganismus, hier also dem jeweiligen Pilz, beimpfte Agarplatte verwendet und dem Wirkstoff ermöglicht, langsam in das Agarmedium zu diffundieren. Dies geschieht zum Beispiel indem ein kleiner mit dem Wirkstoff imprägnierter Rundfilter mit dem Agar in Kontakt gebracht wird, indem er z.B. einfach auf die Platte gelegt wird. Beim Diffundieren des Wirkstoffes durch das mit dem Pilz beimpfte Medium nimmt die Konzentration mit der zurückgelegten Strecke quadratisch ab. Nach einer bestimmten Diffusionsstrecke ist der Wirkstoff dann so verdünnt, dass er keine Wirkung mehr gegen den Pilz zeigt. Die Wirksamkeit einer bestimmten Substanz zeigt sich in der Entstehung von Zonen, in denen das Wachstum inhibiert ist. Diese Zonen sind als klare Bereich um den Punkt zu erkennen, von wo aus der Wirkstoff in das Agarmedium eingedrungen ist. Der Durchmesser dieser Zonen oder Ringe kann gemessen und als Maß für die Wirksamkeit der Substanz herangezogen werden.

Neben dem genannten Test zur Beurteilung der fungiziden Wirksamkeit der 26S/20S Proteasomen-Inhibitoren *in vivo* können auch andere, dem Fachmann bekannte Verfahren verwendet werden, solange sie die Beurteilung einer fungiziden oder fungistatischen Wirkung der getesteten Verbindungen ermöglichen.

Die durch das erfindungsgemäße Verfahren identifizierten Verbindungen können zur Verwendung im Pflanzenschutz in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im Allgemeinen zwischen 0,1 und 95 Gewichtsprozent Wirkstoff, vorzugsweise, zwischen 0,5 und 90 %.

Die Wirkstoffe bzw. ihre Formulierungen können direkt mit den zu bekämpfenden Pilzen oder deren Lebensraum in Kontakt gebracht werden.

### Beispiele

### Beispiel 1

### Herstellung des Proteasomen-Extraktes aus S. cerevisiae-Zellen

150 ml stichfeste Fermenterhefe wurde mit 250 ml Puffer (50 mM Tris-HCl, pH 7,5; 10 mM EDTA) zu 400 ml einer Hefesuspension resuspendiert. Die Zellen wurden in einem Hochdruckhomogenisator bei einem Druck von 1800 psi und 27°C in drei Durchläufen aufgeschlossen. Nach dem Aufschluss der Zellen wurde der pH-Wert mit 0,1M NaOH auf 7,5 eingestellt. Direkt im Anschluss daran erfolgte die Inkubation bei 60°C für 60 Minuten. Darauf wurde die Suspension dreimal bei 13000 rpm in einem JA20 Rotor bei 4°C zentrifugiert, um die ausgefallenen Bestandteile zu entfernen. Der verbleibende Überstand, in dem sich die Proteasomen befinden, konnte in einer 1:100-Verdünnung unmittelbar im Assay zur Identifizierung von Inhibitoren verwendet werden.

Da die Aktivität je nach Fermentationsstadium der Zellen variieren kann, enpfielt es sich, die Aktivität der Proteasomen vorher zu bestimmen, um die geeignete Verdünnung wählen zu können.

### Beispiel 2

### Herstellung des Proteasomen-Extraktes aus U. maydis-Zellen.

50 ml einer *U. maydis*-Kultur wurden der Protoplastierung nach Schulz et al. (Schulz et al. (1990), The *b* alleles of *Ustilago maydis,* whose combinations program pathogenic development, code for polypeptides containing a homeodomain-related motif. *Cell 60,* 295-306) unterworfen. Es folgte die Pelletierung der Protoplasten für 5 min. bei 2000 rpm. Anschließend wurden die Protoplasten durch Zugabe von 50 µl Puffer (50 mM Tris-HCl, pH 7,5; 10 mM EDTA) durch die geänderten osmotischen Bedingungen zum Platzen gebracht. Die Zellreste wurden durch Zentrifugation für 5 min. bei 13 000 rpm pelletiert. Der Überstand wurde wieder für 1 h bei 60°C inkubiert, gefolgt von der Pelletierung der denaturierten Proteine für 5 min. bei 13000 rpm. Der folgende Aktivitätstest der erhaltenen *U. maydis* Proteasomen bestimmt die für den anschließenden Assay zu verwendende Verdünnung.

### Beispiel 3

### Kontrolle des Testsystems mit dem bekannten Inhibitor MG-132 (Aktivitätstest)

Der Aktivitäts- und Inhibitionstest unter Verwendung des bekannten Inhibitores MG-132 von Calbiochem, der zur Kontrolle der Einsatzfähigkeit des erfindungsgemäßen Verfahren dienen soll, wurde in 384er MTPs (Mikrotiterplatten mit 384 Wells) durchgeführt. In einem Gesamtvolumen von 50 µl pro Well waren 25 µl Enzympuffer (10 mM Tris-HCl, 2 mM EDTA) enthaltend die Proteasomen (2,8 mg), 20 µl Substratlösung (13 µM Suc-LLVY-AMC, 0,06% SDS, 10% DMSO, 10 mM Tris-HCl, pH 7,5; 2 mM EDTA) enthaltend das Substrat und 5 µl der Prüfsubstanz (160 nN MG-132 in 5% DMSO) enthalten. Die Vormessung (Fluoreszenz) erfolgte bei 360/465 nm (35 nm Bandbreite). Die Inkubation wurde für 40 Minuten bei 25°C durchgeführt. Die Messung der Fluoreszenz erfolgte wieder bei 360/465 nm (35 nm Bandbreite).

### Beispiel 4

### Identifizierung von Inhibitoren des Proteasoms (Screening)

Für den Proteasomen-Hemmtest im UHTS (Screening) wurden 384er MTPs von Greiner verwendet. Die Prüfsubstanzen wurden in 5 µl Prüfsubstanz-Lösung (50 mM Tris-HCl, pH 7,5; 10 mM EDTA) vorgelegt, so dass im Hemmtest die Endkonzentration der Prüfsubstanzen 2 µM betrug. Auf die Platte wurden in Extra-Wells eine Negativkontrolle von 5 µl (160 nM MG-132; 5% DMSO) sowie eine Positivkontrolle von 5 µl (5% DMSO) aufgetragen. Zur Prüfsubstanzlösung wurden 20 µl der Substratlösung zugegeben (13 µM Suc-LLVY-AMC; 0,06 % SDS; 10 % (v/v) DMSO; 10 mM Tris-HCl, pH 7,5; 2 mM EDTA). Anschließend wurden 25 µl Enzymlösung (10 mM Tris-HCl, pH 7,5; 2 mM EDTA; 280 ng Proteasomen pro Well) zugegeben und die relative Fluoreszenz bestimmt (Vormessung bei 360/465 nm, Bandbreite 25 nm). Es wurde für 40 min. bei 25°C inkubiert. Die Messung der relativen Fluoreszenz erfolgte wiederum bei 360/465 nm, Bandbreite 25 nm.

### Beispiel 5

### Identifizierung von Inhibitoren des Proteasoms (Screening)

Für den Proteasomen-Hemmtest im UHTS (Screening) wurden 384er MTPs von Greiner verwendet. Die Prüfsubstanzen wurden in 5 µl Prüfsubstanz-Lösung (50 mM Tris-HCl, pH 7,5; 10 mM EDTA) in 352 Wells vorgelegt, so dass im Hemmtest die Endkonzentration der Prüfsubstanzen 2 µM betrug. Auf die Platte wurden in Extra-Wells für eine Negativkontrolle (32 Wells) sowie eine Positivkontrolle (32 Wells) je 5 µl 5 % DMSO aufgetragen. Zur Prüfsubstanzlösung wurden 20 µl der Substratlösung zugegeben (13 µM Suc-LLVY-AMC; 0,06 % SDS; 10 % (v/v) DMSO; 10 mM Tris-HCl, pH 7,5; 2 mM EDTA). Anschließend wurden 25 µl Enzymlösung (10 mM Tris-HCl, pH 7,5; 2 mM EDTA; Proteasomenextrakt 1:100 entsprechend 280 ng/Well) zugegeben, wobei für die Negativkontrolle nur Puffer ohne Enzym zugegeben wurde. Anschließend wurde zunächst in einer Vormessung die Fluoreszenz bei einer Anregungswellenlänge von 360 nm und einer Emissionswellenlänge von 460 nm bestimmt (Vormessung bei 360/465 nm, Bandbreite 25 nm). Dann wurden die MTPs bei 37°C für mindestens 40 min inkubiert und anschließen die Fluoreszenz wie zuvor beschrieben bestimmt.

### Beschreibung der Abbildungen

### Abbildung 1:

Aktivitätstest für das 20s Proteasom aus Hefe. Als Substrat für die chymotryptische Aktivität wurde Suc-LLVY-AMC eingesetzt. Die Freisetzung von AMC wurde fluorometrisch über die Zeit bestimmt. Die Proteasomen-Aktivität wurde in einer 1:100 Verdünnung aus einem Hefeaufschluss bei Anwesenheit von 40 nM MG132 und bei Abwesenheit von MG-132 bestimmt. Als Negativ-Kontrolle wurde die Reaktion ohne Enzym durchgeführt (Puffer).

### Abbildung 2

Aktivitätstest für das 20S Proteasom aus *Ustilago maydis.* Als Substrat für die chymotryptische Aktivität wurde Suc-LLVY-AMC eingesetzt. Die Freisetzung von AMC wurde fluorometrisch über die Zeit bestimmt. Die Proteasomen Aktivität wurde in einer 1:100 und 1:20 Verdünnung aus einer *U*. *maydis* Präparation bestimmt. Zudem wurde der höchsten Proteasomenkonzentration der Proteasomen-Inhibitor MG-132 in einer Konzentration von 40 nM zugesetzt.

### Abbildung 3

Aktivitätstest für das 20S Proteasom aus *Botrytis cinerea.* Für die Isolierung des 20S Proteasoms aus *B. cinerea* (*B.c.* Proteasom) wurden Pilzzellen protoplastiert. Die Protoplasten wurden analog zum Protokoll für die Isolierung des 20S Proteasoms aus *U. maydis* weiterverarbeitet (vgl. Bsp. 2). Die chymotryptische Aktivität des *B.c.* Proteasoms wurde anhand der Umsetzung von Suc-LLVY-AMC bestimmt (vgl. Bsp. 3). (x) = 20S Proteasom aus *B. cinerea,* 1:100 verdünnt. (□) = 20S Proteasom aus *S. cerevisiae,* 1:100 verdünnt. (▲) = 20S Proteasom aus *B. cinerea,* 1:500 verdünnt. (◊) = Reaktion ohne Proteasomen.

### Abbildung 4

K_{M}-Wert Bestimmung für das Peptidsubstrat Suc-LLVY-AMC. Eine gleichbleibende Menge von Proteasomen (ca. 70 ng pro Reaktion) wurde mit steigenden Mengen von Substrat inkubiert und anschließend die Zunahme der Fluoreszenz über einen Zeitraum von 5 Minuten bestimmt. Die Steigungen der Kurve (gepunktete Linie) wurden durch die Berechnung von Trendlinien ermittelt. Die Berechnung des K_{M}-Wertes für Suc-LLVY-AMC nach Lineweaver-Burke ergab einen Wert von 5 µM.

### Abbildung 5

Abhängigkeit der Reaktion von der eingesetzten Menge an DMSO. Die Aktivität des 20S Proteasoms aus Hefe wurde anhand der Freisetzung von AMC über die Zeit bestimmt. Der Reaktion wurden steigende Mengen an DMSO zugesetzt. In Abbildung A wurde die Hälfte der Proteinmenge im Vergleich zur Abbildung B eingesetzt. 5 % DMSO sind für die Reaktion insbesondere günstig.

### Abbildung 6

Die Abhängigkeit der Reaktion von der eingesetzten Menge an DMSO wurde wie unter Abbildung 5 beschrieben bestimmt. Die Komponenten aus der Reaktion in Abbildung 5(B) wurden jedoch 72 Stunden bei 4°C gelagert. In Anwesenheit von 5 % DMSO ist die Reaktion auch nach 72 Stunden stabil. Höhere Mengen an DMSO führen zum Verlust der Aktivität.

### Abbildung 7

Aktivitätsnachweis des 20S Proteasoms in 384 well MTPs. Nach 40, 50 und 60 Minuten wurde die Fluoreszenz von AMC in je 192 Wells bestimmt. "PosKo" entspricht der Reaktion mit Enzym, "negKo" entspricht der Reaktion ohne Enzym, "delta" ist die Differenz beider Werte. Bereits nach 40 min. wurde ein z-Faktor > 0,7 erreicht. Der z-Faktor ist eine mathematische Größe zur Bewertung der Uniformität der erhobenen Daten und erlaubt damit eine Bewertung der Verwendbarkeit eines Assays für ein Screening von Substanzen. Der z-Faktor berechnet sich wie folgt: z-Faktor = 1-((3 x Standardabweichung posKo + 3 x Standardabweichung negKo) / (Mittelwert posKo - Mittelwert negKo)), ( Zhang et al. (1999), A Simple Statistical Parameter for Use in Evaluation and Validation of High Throughput Screening Assays. *J. Biomol. Screen. 4(2)*, 67-73).

### Literatur

- 1.: Bogyo, M., Shin, S., McMaster, J. S. & Ploegh, H. L. (1998), *Chem. Biol. 5,* 307-320.
- 2.: Coux et al. (1995), *Ann. Rev. Biochem. 65,* 801-847.
- 3.: Coux et al. (1998), *J. Biol. Chem. 273*(15), 8820-8828.
- 4.: Driscoll und Goldberg (1990), *J*. *Biol. Chem.* 265, 4789.
- 5.: EP 0 345 750
- 6.: Fenteany et al. (1995), *Science, 268,* 726-731.
- 7.: Groll et al. (1997), *Nature 386,* 463-471.
- 8.: JP 05292964
- 9.: JP 06022759
- 10.: Klafky et al. (1995), *Neuroscience Letters 201,* 29-32.
- 11.: Knowlton et al. (1997), *Nature 390,* 639-643.
- 12.: Kroll et al. (1999), *Chem. Biol. 6,* 889-698.
- 13.: Lee und Goldberg (1989), *J. Biol. Chem. 271,* 27280.
- 14.: Lee und Goldberg (1998), *Molecular and Cellular Biology 18,* 30-38.
- 15.: McCormack et al. (1998), *Biochemistry 37,* 7792-7800.
- 16.: Mellgren (1997), *J Biol. Chem. 272(47),* 29899-29903.
- 17.: Meng et al. (1999), *PNAS 96,* 10403.
- 18.: Mitchell und Carter (2000), *Bioscene 26(3),* 9-13.
- 19.: Orlowski et al. (1993), *Biochemistry 32,* 1563.
- 20.: Schulz et al. (1990), *Cell 60,* 295-306.
- 21.: Stack et al. (2000), *Nature Biotechnology 18,* 1298-1302.
- 22.: Stein et al. (1996), *Biochemistry 35*, 3899.
- 23.: WO 00/23614
- 24.: WO 00/33654
- 25.: WO 00/43000
- 26.: WO 91/13904
- 27.: WO 95/24914
- 28.: WO 98/42829
- 29.: Zhang et al. (1999), *J*. *Biomol. Screen. 4(2)*:67-73).

## Patentansprüche

1. Verfahren zum Identifizieren von Fungiziden durch Testen einer Kandidatenverbindung in einem 20S-Proteasomen-Hemmtest.

2. Verfahren gemäß Anspruch 1, **dadurch gekennzeichnet, dass** man 20S Proteasomen aus Pilzen verwendet.

3. Verfahren gemäß Anspruch 1 oder 2, **dadurch gekennzeichnet, dass** die Wirkung der ausgewählten Kandidatenverbindungen gegenüber Pilzen anschließend in einem *in vivo* Test überprüft wird.

4. Verfahren gemäß einem der Ansprüche 1 bis 3, **dadurch gekennzeichnet, dass** man
a) eine Kandidatenverbindung mit 20S Proteasomen bei Anwesenheit eines Substrats und bei Anwesenheit von 2 bis 10 % (v/v) Dimethylsulfoxid in Kontakt bringt, und
b) diejenigen Kandidatenverbindungen auswählt, die die enzymatische Umsetzung des Substrats durch die 20S Proteasomen spezifisch inhibieren.

5. Verfahren gemäß Anspruch 4, **dadurch gekennzeichnet, dass** das Substrat eine Gruppe enthält, deren Freisetzung durch eine enzymatische Aktivität des 20S Proteasoms fluorimetrisch oder colorimetrisch nachgewiesen werden kann.

6. Verfahren gemäß Anspruch 4 oder 5, **dadurch gekennzeichnet, dass** 20S Proteasomen verwendet werden, die isoliert wurden, indem man
a) eukaryontische Zellen aufschließt,
b) die entstandene Zellsuspension auf 50°C bis 70°C erhitzt, bis die temperatursensitiven Bestandteile ausgefallen sind, und
c) die Proteasomen durch Abzentrifugieren der unlöslichen Bestandteile gewinnt.

## Claims

1. Method for identifying fungicides by assaying a candidate compound in a 20S-proteasome inhibition assay.

2. Method according to Claim 1, **characterized in that** 20S proteasomes from fungi are used.

3. Method according to Claim 1 or 2, **characterized in that** the action of the selected candidate compounds on fungi is subsequently checked in an *in-vivo* assay.

4. Method according to one of Claims 1 to 3, **characterized in that**
a) a candidate compound is contacted with 20S proteasomes in the presence of a substrate and in the presence of from 2 to 10% (v/v) dimethyl sulphoxide, and
b) those candidate compounds which specifically inhibit the enzymic conversion of the substrate by the 20S proteasome are selected.

5. Method according to Claim 4, **characterized in that** the substrate comprises a group whose liberation by an enzymic activity of the 20S proteasome can be detected fluorimetrically or colorimetrically.

6. Method according to Claim 4 or 5, **characterized in that** 20S proteasomes are used that have been isolated by
a) disrupting eukaryotic cells,
b) heating the cell suspension generated to from 50°C to 70°C, until the temperature-sensitive components have precipitated, and
c) recovering the proteasomes by removing the insoluble components by means of centrifugation.

## Revendications

1. Méthode d'identification de fongicides par épreuve d'un composé candidat dans un test d'inhibition de protéasomes 20S.

2. Méthode suivant la revendication 1, **caractérisée en ce qu'**on utilise des protéasomes 20S issus de champignons.

3. Méthode suivant la revendication 1 ou 2, **caractérisée en ce que** l'action des composés candidats choisis vis-à-vis de champignons est ensuite contrôlée dans un test in vivo.

4. Méthode suivant l'une des revendications 1 à 3, **caractérisée en ce que** :
a) on met en contact un composé candidat avec des protéasomes 20S en présence d'un substrat et en présence de 2 à 10 % (en volume/volume) de diméthylsulfoxyde, et
b) on choisit les composés candidats qui inhibent spécifiquement la transformation enzymatique du substrat par les protéasomes 20S.

5. Méthode suivant la revendication 4, **caractérisée en ce que** le substrat contient un groupe dont la libération par une activité enzymatique du protéasome 20S peut être mise en évidence par fluorimétrie ou par colorimétrie.

6. Méthode suivant la revendication 4 ou 5, **caractérisée en ce qu'**on utilise des protéasomes 20S, que l'on a isolés
a) en désagrégeant des cellules eucaryotiques,
b) en chauffant à une température de 50°C à 70°C la suspension cellulaire produite, jusqu'à ce que les constituants sensibles à la chaleur aient précipité, et
c) en isolant les protéasomes par centrifugation des constituants insolubles.
